# EUROPEAN PATENT SPECIFICATION

(11) **EP 2 402 012 B1**
(45) Date of publication and mention of the grant of the patent: **19.12.2012**
(21) Application number: 11172101.5
(22) Date of filing: 30.06.2011
(51) Int. Cl.: A61K 31/4545, A61K 9/08, A61P 11/02, A61P 11/04, A61P 37/08

(54) **Liquid formulations of rupatadine fumarate**
Flüssigformulierungen von Rupatadinfumarat
Formulations liquides pour fumarate de rupatadine

(30) Priority: 16.07.2010 US 364992 P; 30.06.2010 EP 10382184
(43) Date of publication of application: 04.01.2012
(73) Proprietor: J. Uriach y Compania S.A., 08184 Palau-solità i Plegamans (Barcelona) (ES)
(72) Inventor: Suriol Ferrer, M Montserrat, E-08018 Barcelona (ES); Morlesín Capdevila, M Silvia, E-08020 Barcelona (ES)
(74) Representative: ABG Patentes, S.L.

(56) References cited:
- EP-A1- 0 577 957
- WO-A2-2006/131737
- ES-A1- 2 087 818
- DATABASE WPI Week 201031 Thomson Scientific, London, GB; AN 2010-D58323 XP002604402, -& CN 101 669 926 A (DONGGUAN TAILI BIOTECH CO LTD) 17 March 2010 (2010-03-17) -& CN 101 669 926 A (DONGYING TAILI BIOLOG ENGINEER) 17 March 2010 (2010-03-17)
- DATABASE WPI Week 201111 Thomson Scientific, London, GB; AN 2011-B22141 XP002658722, -& CN 101 926 762 A (UNIV SOUTHEAST) 29 December 2010 (2010-12-29)

## Description

### Field of the invention

The present application relates to cyclodextrin-free aqueous liquid formulations of rupatadine fumarate.

### Background of the invention

Rupatadine (I) is an authorized antihistaminic agent and has IUPAC name 8-Chloro-6,11-dihydro-11-[1-[(5-methyl-3-pyridinyl)methyl]-4-piperidinylidene]-5H-benzo[5,6]cyclohepta[1,2-b]pyridine, CAS number 158876-82-5 for the free base and the following chemical formula:

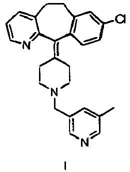

Rupatadine is currently marketed in 10 mg (rupatadine) tablets as rupatadine fumarate (CAS 182349-12-8 for the fumarate salt) for the treatment of allergic rhinitis and urticaria in adults and teenagers.

Rupatadine free base was first disclosed in EP0577957.

Spanish patent application ES2087818 discloses the monofumarate salt of rupatadine (i.e. rupatadine fumarate) and aqueous liquid pharmaceutical compositions of rupatadine fumarate. In particular, this document discloses a syrup containing rupatadine fumarate at 4 g/L, sucrose, a flavouring agent, a sweetening agent and water; and a suspension for injection which contains rupatadine fumarate at 20 g/L, benzyl alcohol, propyleneglycol and water.

EP0577957 discloses some liquid pharmaceutical compositions of rupatadine free base; compound **4** in EP0577957 is rupatadine free base. The formulations disclosed therein are identical to those disclosed in ES2087818 but rupatadine free base is used instead of rupatadine fumarate.

Despite the aqueous liquid pharmaceutical compositions disclosed in EP0577957 and ES2087818, the inventors have found that the solubility in water of rupatadine fumarate is 2.9 g/L (see Reference example 1) and therefore these prior art formulations may have stability problems due to supersaturation of rupatadine free base or rupatadine fumarate and would not be suitable for use as a medicament.

CN101669901 and CN101669926 disclose liquid formulations of rupatadine free base using cyclodextrins to dissolve rupatadine.

CN101669901 is directed to liquid formulations of rupatadine free base for ophthalmic delivery comprising rupatadine, a solvent and a cyclodextrin.

CN10169926 is directed to liquid formulations of rupatadine free base for nasal delivery comprising rupatadine, a solvent and a cyclodextrin. It is stated that rupatadine has low solubility in water (1.39 mg/mL to 0.82 mg/mL at pH 3.0 to 7.0, table 9 in CN10169926) and the problem of its low solubility is solved using cyclodextrins (tables 10-12 of CN10169926) in order to obtain liquid formulations.

CN101926762, which was published after the earliest priority date of the present application, is directed to a stable eye drop formulation that comprises rupatadine fumarate, a thickener, a buffer salt, an isotonic agent, an antibacterial agent, a solubilizer and water.

WO 2006/131737 discloses homogeneous pharmaceutical compositions comprising an antihistaminic agent, a polar liposome and a pharmaceutically acceptable aqueous carrier.

Due to the above, it is desirable to provide cyclodextrin-free aqueous liquid formulations of rupatadine fumarate.

### Brief description of the invention

The present invention relates to a cyclodextrin-free aqueous liquid pharmaceutical composition comprising:
- rupatadine fumarate,
- one or more cosolvents and
- one or more pH regulating agents selected from the group consisting of buffers and bases wherein the composition has a pH between 4 and 6.5.

Another embodiment of the invention is a process to prepare a composition according to claim 1 which comprises:
a) dissolving rupatadine fumarate in the one or more cosolvents to yield a first solution, and
b) adding water and the one or more pH regulating agents selected from the group consisting of buffers and bases to the first solution.

Another embodiment of the invention is a process to prepare a composition according to claim 1 when the pH regulating agent is a buffer which comprises:
a) dissolving rupatadine fumarate in the one or more cosolvents to yield a first solution,
b) dissolving the one or more pH regulating agents selected from the group consisting of buffers and bases in water to yield a second solution, and
c) mixing the first and second solutions obtained in steps a) and b).

Another embodiment of the invention is a process to prepare a composition according to claim 1 when the pH regulating agent is a base which comprises:
a) dissolving rupatadine fumarate in the one or more cosolvents,
b) adding water to the previous solution, and
c) adding the base.

Another embodiment of the present invention is a cyclodextrin-free aqueous liquid composition comprising:
- rupatadine fumarate,
- one or more cosolvents and
- one or more pH regulating agents selected from the group consisting of buffers and bases
wherein the composition has a pH between 4 and 6.5 for use in the treatment of allergic conditions.

Another embodiment of the present invention is the use of a cyclodextrin-free aqueous liquid composition comprising:
- rupatadine fumarate,
- one or more cosolvents and
- one or more pH regulating agents selected from the group consisting of buffers and bases wherein the composition has a pH between 4 and 6.5 for the treatment of allergic conditions.

Another embodiment is a method of treating an allergic condition which comprises administering to a subject in need thereof, preferably a human being, a cyclodextrin-free aqueous liquid composition comprising:
- rupatadine fumarate,
- one or more cosolvents and
- one or more pH regulating agents selected from the group consisting of buffers and bases wherein the composition has a pH between 4 and 6.5.

### Definitions

As used herein, a cosolvent is a pharmaceutically acceptable organic solvent which is soluble in water (which dissolves at least one part of the solvent in two parts of water) and improves (*i.*e. increases) the solubility of the solute (rupatadine fumarate in this context) in water. Examples of cosolvents include monohydric alcohols (e.g. ethanol, isopropanol), polyhydric alcohols or polyols (e.g. propylene glycol, glycerine), ethers (e.g. macrogols such as macrogol 300 or macrogol 400) and substituted amides (e.g. dimethylacetamide and pyrrolidone).

As used herein "cosolvent(s)" is equivalent to "one or more cosolvents".

As used herein "pH regulating agent(s)" is equivalent to "one or more pH regulating agents".

As used herein "buffer(s)" is equivalent to "one or more buffers".

As used herein the term sweetener designates any natural or artificial substance capable of imparting sweet taste to aqueous solutions or dispersions.

As used herein a sugar is a carbohydrate of formula (CH₂O)ₙ wherein n is comprised between 5 and 12 (i.e. a monosaccharide or a disaccharide). Sugars (such as sucrose and fructose, glucose) are capable of imparting a sweet taste to aqueous solutions or dispersions.

As used herein, a pH regulating agent is an agent that can change (decrease or increase) the pH of a solution or stabilize the pH of a solution at a given pH, *e.g*. an acid, a base or buffer. Therefore the pH regulating agent can change or stabilize the pH of a solution of rupatadine fumarate.

A pharmaceutically acceptable organic solvent is an organic solvent which is authorised to be used in pharmaceutical preparations by the regulatory authorities. The pharmaceutically acceptable solvents are well known by the skilled person in the art; see e.g. Handbook of pharmaceutical excipients, APhA Publications 5th edition 2005, edited by Raymond C. Rowe, Paul J. Sheskey, Siân C. Owen, ISBN-10: 1582120587.

The term "pharmaceutically acceptable salt" refers to those salts which are, according to medical judgement, suitable for use in contact with the tissues of humans and other mammals without undue toxicity, irritation and allergic response. Pharmaceutically acceptable salts are well known in the art.

An acid is a substance that releases hydrogen ions and decreases the pH of a solution. A strong acid is an acid that when dissolved in water is completely ionized into hydrogen ions and the corresponding anion. A weak acid is a substance that when dissolved in water is only partially ionized into hydrogen ions and anions. Examples of strong acids include: HCl, H₂SO₄, HNO₃ and p-toluenesulphonic acid.

Examples of weak acids include CH₃COOH, H₃PO₄, citric acid and oxalic acid.

A base is a substance that can accept hydrogen ions and increases the pH of a solution. A strong base is a base which when dissolved in water accepts the same amount (in mol) of hydrogen ions as of base dissolved. A weak base is a base that when dissolved in water accepts only a fraction (in mol) of the base dissolved. Examples of strong bases are: NaOH, KOH and LiOH. Examples of weak bases are NH₃, pyridine, methylamine, triethylamine, and NaHCO₃.

The pKa is a measurement of the strength of an acid. The lower the pKa, the stronger the acidity. The higher the pKa, the weaker the acid. The pKb is a related measurement and is a measurement of the strength of a base; the lower the pKb, the stronger the base and the higher the pKb, the weaker the base.

A conjugate base of an acid is the anion of an acid when the acid has released a hydrogen ion, e.g. the conjugate base of H₃PO₄, is H₂PO₄⁻. The conjugate acid of a base is the cation formed when the hydrogen ion is accepted, *e.g*. the conjugate acid of NH₃, is NH₄⁺.

In the present invention the term buffer is used to designate either a) a mixture of a weak acid and its conjugate base, b) a mixture of a weak base and its conjugate acid, c) a mixture of a weak base and a conjugate acid of a different base or d) a mixture of a weak acid and a conjugate base of a different acid. The term buffer solution is meant to designate a solution in water of a buffer. When small amounts of acids or bases are added to buffer solutions, the pH is maintained nearly constant. A buffer solution of an acid and its conjugate base in the same molar amount results in a pH equal to the pKa of the acid. As used herein, buffers are indicated as X/Y, which means the buffer is a mixture of components X and Y; for example the buffer Na₂HPO₄/citric acid means it is a mixture of Na₂HPO₄ and citric acid.

As used herein a surfactant is an amphiphilic compound that lowers the surface tension of a liquid, the interfacial tension between two liquids or that between a liquid and a solid.

An acidic solution is a solution with acid pH, *i.e*. a pH lower than 7. A basic solution is a solution with basic pH, *i.e*. a pH higher than 7.

In the context of the present invention when rupatadine is mentioned it is intended to be considered as free base, when rupatadine fumarate is mentioned it is referred to said salt. In the present invention the concentrations of rupatadine fumarate solutions are expressed by reference to the corresponding amount of rupatadine free base (and are indicated as g (rupatadine)/L). Thus, to convert the concentrations of rupatadine fumarate expressed as g (rupatadine)/L to concentrations expressed as g (rupatadine fumarate)/L the values need to be multiplied by a factor of 1.28.

As used herein, the terms pharmaceutical composition, composition and formulation are used interchangeably.

Unless otherwise indicated, all the experimental data and procedures described herein are obtained or performed at room temperature and atmospheric pressure.

Unless otherwise indicated, all concentrations indicated as % correspond to % w/v. Thus, a solution containing 1% of a solute means it contains 1 g(solute)/100 mL.

As used herein, when ranges are given it is intended to cover all the intermediate values as well as the end values. Thus, for example, when it is stated that "the pH is between 4.5 and 5.5", the pH can have any value between 4.5 and 5.5, including the end values 4.5 and 5.5.

### Detailed description of the invention

The inventors have found that rupatadine fumarate in acidic solutions forms an adduct (Compound II) of rupatadine and fumaric acid.

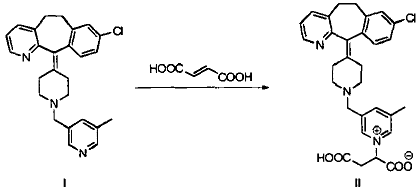

Considering this, aqueous acidic pharmaceutical compositions of rupatadine fumarate are not suitable when formulating a medicament for the administration of rupatadine fumarate because Compound II will develop upon storage as an impurity at excessive levels. On the other hand, the solubility of rupatadine fumarate is pH dependent and the solubility is high at acidic pH and decreases when the pH raises (see Reference example 1); therefore aqueous pharmaceutical compositions comprising high levels of rupatadine fumarate at basic pH are also not possible because rupatadine fumarate is not sufficiently soluble.

According to the liquid pharmaceutical compositions of rupatadine and rupatadine fumarate disclosed in the art, those problems were not evident to the person skilled in the art.

The inventors have found that the above problems can be solved when formulating a cyclodextrin-free aqueous liquid pharmaceutical composition of rupatadine fumarate comprising:
- rupatadine fumarate,
- one or more cosolvents and
- one or more pH regulating agents selected from the group consisting of buffers and bases wherein the composition has a pH between 4 and 6.5.

In one embodiment the aqueous liquid pharmaceutical compositions of the present invention are solutions.

In an embodiment the total amount of cosolvent(s) is from 5 to 50 % of the formulation. In another embodiment the total amount of cosolvent(s) is from 5 to 25 % of the formulation.

In an embodiment, the composition comprises one or more cosolvents selected from the group consisting of monohydric alcohols, polyhydric alcohols or polyols, ethers and substituted amides.

In another embodiment, the one or more cosolvents are selected from the group consisting of ethanol, isopropanol, propylene glycol, glycerine, macrogol 300, macrogol 400, dimethylacetamide and pyrrolidone.

In an embodiment, the one or more cosolvents are selected from the group consisting of ethanol, propylene glycol, macrogol 300 and macrogol 400. In another embodiment, the one or more cosolvents are selected from the group consisting of propylene glycol, macrogol 300 and macrogol 400.

In another embodiment, the composition comprises only one cosolvent. In another embodiment the composition comprises only one cosolvent selected from the group consisting of ethanol, propylene glycol, macrogol 300 and macrogol 400. In another embodiment the composition comprises only one cosolvent selected from the group consisting of propylene glycol and macrogol 400. In another embodiment the composition comprises only one cosolvent which is propylene glycol. In another embodiment the composition comprises only one cosolvent which is a macrogol, preferably macrogol 300 or macrogol 400. In another embodiment, the composition comprises only one cosolvent which is macrogol 300. In another embodiment, the composition comprises only one cosolvent which is macrogol 400.

It has also been found that the compositions of the present invention are particularly stable (in terms of formation of Compound II upon storage) when the cosolvent is not a surfactant, particularly when it is not a polysorbate.

In an embodiment, the cosolvent(s) do not comprise a surfactant.

In another embodiment the cosolvent(s) do not comprise a surfactant and the total amount of cosolvent(s) is from 5 to 50 % of the formulation. In another embodiment the cosolvent(s) do not comprise a surfactant and the total amount of cosolvent(s) is from 5 to 25 % of the formulation.

The pH regulating agent(s) in the pharmaceutical compositions of the invention can be buffers, bases, or mixtures thereof.

In another embodiment, the one or more pH regulating agents are selected from the group consisting of buffers. The buffer(s) are used to adjust the pH of the composition in the range of 4-6.5 and, to this effect, they preferably have a pKa in the range of 4-6.5. In another embodiment the buffer(s) show a pKa in the range of 4-6. In another embodiment the buffer(s) show a pKa in the range of 5-6.5. In a further embodiment the buffer(s) show a pKa in the range of 5-6. In a further embodiment the buffer(s) show a pKa in the range of 4.5-5.5. In another embodiment the buffer(s) show a pKa in the range of 4.5-6. Examples of suitable buffers are: phosphate buffers (such as K₂HPO₄/KH₂PO₄, Na₂HPO₄/NaH₂PO₄, Na₂HPO₄/KH₂PO₄), Na₂HPO₄/citric acid, citrate buffers, acetate buffers (such as acetic acid/sodium acetate, acetic acid/ammonium acetate), and ammonium acetate/sodium edetate. These and further buffers can be found in the buffer solutions section of the European Pharmacopoeia (4.1.3). In an embodiment, the composition comprises only one buffer. In an embodiment, the composition comprises only one buffer which is Na₂HPO₄/citric acid, Na₂HPO₄/KH₂PO₄ or Na₂HPO₄/NaH₂PO₄. In an embodiment the composition comprises only one buffer which is Na₂HPO₄/citric acid or Na₂HPO₄/KH₂PO₄. In another embodiment the composition comprises only one buffer which is Na₂HPO₄/NaH₂PO₄. In another embodiment the composition comprises only one buffer which is Na₂HPO₄/citric acid. In another embodiment the composition comprises only one buffer which is Na₂HPO₄/KH₂PO₄.

Phosphate buffers are buffers formed with pharmaceutically acceptable salts of HPO₄²⁻ and H₂PO₄⁻. Examples include K₂HPO₄/KH₂PO₄, Na₂HPO₄/NaH₂PO₄ and Na₂HP0₄/KH₂PO₄. Said buffers can be obtained using the specific combination of phosphate salts or any phosphate species (including phosphoric acid) and adjusting the pH with acids or bases.

Citrate buffers are buffers formed using citric acid and/or pharmaceutically acceptable salts of citric acid. Said buffers can be obtained adding the specific citrate species or adding citric acid or citrate salts and adjusting the pH with acids or bases.

Acetate buffers are buffers formed using acetic acid and/or pharmaceutically acceptable salts of acetic acid such as sodium acetate or ammonium acetate. Said buffers can be obtained adding the specific acetate species or adding acetic acid or acetate salts and adjusting the pH with acids or bases.

In an embodiment the one or more pH regulating agents are selected from the group consisting of bases. Suitable bases are: NaOH and KOH. In an embodiment, the composition comprises only one base. In an embodiment the composition comprises only one base which is NaOH.

In another embodiment the one or more pH regulating agents are selected from the group consisting of phosphate buffers, Na₂HPO₄/citric acid, citrate buffers, acetate buffers, ammonium acetate/sodium edetate and NaOH.

In an embodiment the total amount of the pH regulating agent(s) is from 1 % to 10 %. In an embodiment the total amount of the pH regulating agent(s) is from 1 % to 5 %. In a further embodiment the total amount of the pH regulating agent(s) is from 1 % to 3 %.

In an embodiment the pH of the formulation is between 4 and 6. In another embodiment the pH of the formulation is between 4.5 and 6. In another embodiment the pH of the formulation is between 5 and 6.5. In another embodiment the pH of the formulation is between 5 and 6. In another embodiment the pH of the formulation is between 4.5 and 5.5.

The pharmaceutical compositions of the invention can be used for oral administration.

The pharmaceutical compositions of the invention can be used for nasal administration.

When a pharmaceutical composition of the present invention is intended for nasal administration it is required to be isotonic or slightly hypertonic with the nasal mucosa.

The pharmaceutical compositions of the invention can further comprise one or more of usual pharmaceutically acceptable excipients. Said excipients are well known in the art and comprise sweeteners, preservatives, colorants, flavouring agents and thickening agents.

Suitable excipients and its role can be found on Handbook of pharmaceutical excipients, APhA Publications 5th edition 2005, edited by Raymond C. Rowe, Paul J. Sheskey, Siân C. Owen, ISBN-10: 1582120587. Specially suitable excipients for use in the compositions of the invention are detailed below.

Rupatadine fumarate has a bitter taste and if the liquid pharmaceutical compositions according to the invention are intended for oral use it is desirable that said formulations further comprise a sweetener to mask this bitter taste.

Suitable sweeteners are preferably selected from the group consisting of sugars (such as sucrose, fructose and glucose), artificial sweeteners (such as saccharin or its pharmaceutically acceptable salts (such as saccharin sodium), cyclamate or its pharmaceutically acceptable salts (such as cyclamate sodium), aspartame, acesulphame or its pharmaceutically acceptable salts (such as acesulphame potassium), sucralose, neohespiridin dihydrochalcone and naringin dihydrochalcone), and mixtures thereof.

In an embodiment the sweetener is a sugar, an artificial sweetener or mixtures thereof. In an embodiment the sweetener is sucrose, saccharin or its pharmaceutically acceptable salts (such as saccharin sodium), or mixtures thereof.

It has also been found that the compositions of the present invention comprising a sweetener are particularly stable (in terms of formation of Compound II upon storage) when the sweetener is a sugar or an artificial sweetener, particularly when the sweetener is sucrose, saccharin or its pharmaceutically acceptable salts (such as saccharin sodium) or mixtures thereof. In an embodiment, the composition comprises a sweetener selected from a sugar, an artificial sweetener, and mixtures thereof. In another embodiment, the composition comprises a sweetener selected from sucrose, saccharin or its pharmaceutically acceptable salts, and mixtures thereof.

In another embodiment, the compositions of the invention do not comprise a sweetener.

In another embodiment, the invention relates to compositions which further comprise one or more of the following excipients:
- one or more colorants.
- one or more flavouring agents.
- one or more preservative agents.

Suitable preservatives are methyl parahydroxybenzoate, benzyl alcohol, propyl parahydroxybenzoate, 2,4-dichlorobenzylic alcohol and benzalkonium chloride).

Suitable colorants are red colorant E-123, caramel colour E-150 and quinoline yellow E-104.

Suitable flavouring agents are strawberry flavour, cherry flavour, banana flavour, mint flavour, orange, lemon, vainillin, peppermint and grape.

Suitable thickening agents are xantham gum, carmellose sodium and promellose.

In an embodiment the pharmaceutical composition of rupatadine fumarate of the present invention comprises rupatadine fumarate at a concentration of from 0.5 to 1.5 g(rupatadine)/L. In another embodiment the pharmaceutical composition of rupatadine fumarate comprises rupatadine fumarate at a concentration of 1 g(rupatadine)/L.

Another embodiment of the present the invention is a cyclodextrin-free stable aqueous liquid pharmaceutical composition comprising:
- rupatadine fumarate,
- one or more cosolvents and
- one or more pH regulating agents selected from the group consisting of buffers and bases
wherein the composition has a pH between 4 and 6.5 and wherein the total amount of cosolvent(s) is from 5 to 50 % of the formulation.

Another embodiment of the invention is a cyclodextrin-free stable aqueous liquid pharmaceutical composition comprising:
- rupatadine fumarate,
- one or more cosolvents and
- one or more pH regulating agents selected from the group consisting of buffers and bases wherein the composition has a pH between 5 and 6.

Another embodiment of the invention is a cyclodextrin-free stable aqueous liquid pharmaceutical composition comprising:
- rupatadine fumarate,
- one or more cosolvents and
- one or more pH regulating agents selected from the group consisting of buffers and bases
wherein the composition has a pH between 5 and 6 and wherein the total amount of cosolvent(s) is from 5 to 50 % of the formulation.

Another embodiment of the invention is a cyclodextrin-free stable aqueous liquid pharmaceutical composition comprising:
- rupatadine fumarate,
- one or more cosolvents and
- one or more pH regulating agents selected from the group consisting of buffers and bases
wherein the composition has a pH between 5 and 6 and wherein the one or more cosolvents are selected from the group consisting of propylene glycol, macrogol 300 and macrogol 400.

Another embodiment of the present invention is the cyclodextrin-free aqueous liquid formulations of the present invention for use in the treatment of allergic conditions. In one embodiment the allergic condition is allergic rhinitis or urticaria. In another embodiment the composition of the present invention is used for the treatment of allergic conditions in paediatric population. In another embodiment the allergic condition treated in the paediatric population is allergic rhinitis or urticaria.

### Methods

The following methods are used to determine the pH, the % of Compound II present and the clarity of the formulations described herein.

### Method 1: Method of measuring the pH

The pH is measured according to the European Pharmacopoeia 2.2.3 at room temperature (20-25°C) and atmospheric pressure using a potentiometric pH meter (such as a Crison micropH 2001) apparatus calibrated using a pH 7.00 and 4.01 buffer solutions.

### Method 2: Method of measuring the % Compound II

The HPLC method used to determine the % Compound II present in a formulation is based on the European Pharmacopoeia 2.2.29 using the following conditions:
- room temperature and atmospheric pressure
- column: C1810 µm (3.9 mm x 30 cm)
- eluent: Methanol - Phosphate buffer solution pH 4.2 (75:25)
- eluent flow: 1.3 mL/min
- detection: UV-Visible spectrophotometer at 265 nm
- retention time of Rupatadine: aprox. 8.9 min
- relative retention time of Compound II: aprox. 0.43

Reference standard solutions of rupatadine and Compound II (which can be prepared as described in Reference Example 3) are used in the identification of the components. The test sample is compared to the equivalent placebo (same formulation without rupatadine fumarate) to check overlapping peaks with other excipients in the formulation.

The % Compound II is calculated as 100*(area of Compound II peak)/(area of rupatadine peak).

### Method 3: Method of measuring the clarity of a solution

The clarity of a solution was measured according to the European Pharmacopoeia 2.2.1 as follows at room temperature and atmospheric pressure.

Reference suspensions I-IV (suspension I is the clearest and IV the least clear) were prepared according to the European Pharmacopoeia 2.2.1. The samples of rupatadine fumarate solution to be tested were visually compared to the reference suspensions and their clarity was visually rated in relation to reference suspensions I to IV, *i.e*. a sample with clarity < I means that it is clearer than the clearest reference.

### Method 4: Accelerated stability

The formulations were stored in a climatic chamber at 40°C / 75% Relative Humidity (RH) in closed amber glass vials during a 3 month period. At the end of the 3-m period, the % of Compound II present and the clarity of the solution were measured according to *Method* 2 and *Method* 3 respectively.

Accelerated stability studies allow predicting the stability of a composition in a short term. Typically it is considered that a composition has a desirable stability when after 1 month in the conditions mentioned above it does not have more than 0.45 % Compound II determined using *Method* 2, and preferably when after 1 month in the conditions mentioned above it does not have more than 0.30 % Compound II determined using *Method 2*. Stable compositions typically do not have, after 3 months in the conditions mentioned above, more than 0.85 % Compound II determined using *Method 2.*

The solutions are also tested under *Method 3* to determine its clarity, which is an indication of the presence of precipitated material. Preferably stable solutions are clearer than the clearest reference when evaluated using *Method 3.*

### Reference examples

### Reference example 1: Solubility of rupatadine fumarate in water at different pH

The solubility of rupatadine fumarate was determined by adding 25 g/L of rupatadine fumarate to water and stirring during 16 - 24 h. The resulting solution was filtered and the amount of rupatadine determined using the *Method 2.*

The solubility of rupatadine fumarate in water using the procedure described above is 2.9 g/L.

Using the same procedure, but using buffer solutions of known pH instead of water, the solubility of rupatadine fumarate at different pH was determined (the pH of the solution was measured prior to the addition of rupatadine fumarate).

| **pH** | **Solubility (g(rupatadine fumarate)/L)** |
|---|---|
| 1.4 | 19.60 |
| 2.0 | 7.70 |
| 2.4 | 5.59 |
| 3.1 | 4.86 |
| 4.1 | 4.05 |
| 5.5 | 4.06 |
| 6.0 | 0.96 |
| 6.4 | 0.17 |
| 7.0 | 0.035 |
| 11.0 | <0.005 |

### Reference example 2: Rate of formation of Compound II in aqueous solutions of rupatadine fumarate

It has been found that rupatadine reacts with fumaric acid in acidic conditions to yield an adduct (Compound II):

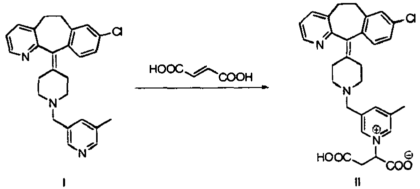

Different aqueous solutions comprising rupatadine fumarate at a concentration of 1 g(rupatadine fumarate)/L at different pH (3.41, 4.38 and 5.00), obtained adding the required amount of solutions of HCl_{(aq)} or NaOH_{(aq)} to solutions containing only water and rupatadine fumarate, were stored at different temperature conditions (room temperature and 40°C/75%HR) during two months and analyzed at the beginning, at one month and at the end of the period using Method 2, see table below.

| **pH** | **% Compound II initial** | **% Compound II 1 month** | | **% Compound II 2 months** | |
|---|---|---|---|---|---|
| | | **25 ± 2°C** | **40 ± 2°C** | **25 ± 2°C** | **40 ± 2°C** |
| 3.41 | 0.035 | 0.174 | 0.737 | 0.276 | 1.396 |
| 4.38 | 0.036 | 0.090 | 0.357 | 0.142 | 0.646 |
| 5.00 | 0.040 | 0.039 | 0.112 | 0.051 | 0.182 |

These results show that the rate of formation of Compound II is pH- dependent. The lower the pH, the higher the rate of formation of Compound II.

### Reference Example 3: Preparation of (Compound II

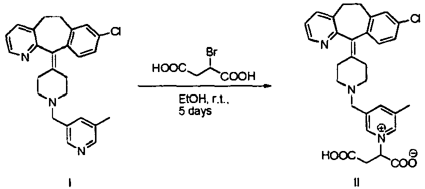

35 g (0.084 mol) of rupatadine (I) were dissolved in 260 mL of ethanol in a 1000 mL flask. To that solution 16.1 g (0.087 mol) of bromosuccinic acid were added and the mixture was allowed to react overnight at room temperature. The mixture was concentrated to half the volume and allowed to react at room temperature during 5 days.

The solvent was removed *in vacuo* and the solid washed with ethanol and with an ethanol:water:ammonia mixture. Next, it was purified by flash chromatography using a 9:1:1 ethanol:ammonia:water mixture.

Finally 4.51 g (0.0085 mol, 9.7 % yield) of the desired product (Compound II) were obtained.

**¹H-NMR** (300 MHz, CD₃OD): 8.71 (wide signal, 2H), 8.31 (m, 2H), 7.63 (d, *J*=7.31 Hz, 1H), 7.18 (m, 4H), 5.48 (dd, *J*=3.29 Hz, *J*=11.70 Hz, 1H), 3.69 (s, 2H), 3.41 (m, 3H), 3.11 (dd, *J*=11.7 Hz, *J*=17.2 Hz, 1H), 2.8 (m, 4H), 2.54 (s, 3H), 2.45 (m, 2H), 2.26 (m, 4H).

**¹³C-NMR** (75.43 MHz, CD₃OD): 176.35, 172.20, 158.73, 147.00, 144.65, 143.31, 143.26, 141.15, 140.03, 139.69, 139.47, 138.65, 138.62, 135.85, 134.06, 133.72, 131.91, 130.33, 127.01, 124.08, 75.81, 59.36, 55.47, 55.42, 42.30, 32.75, 32.03, 31.81, 31.77, 18.38.

IR (KBr): 3412, 1587, 1474, 1437, 1379, 1176, 1086, 992, 874, 829, 663 cm⁻¹.

| | | |
|---|---|---|
| **EA:** | Calculated (C₃₀H₃₀ClN₃O₄·NH₃·H₂O): | C 63.54; H 6.22; N 9.88 |
| | Found: | C 63.47; H 6.41; N 9.61 |

### Examples

### Example 1: Preparation of a nasal liquid formulation of rupatadine fumarate (1.00 g(rupatadine)/L)

The quantitative composition of this formulation is disclosed in the table below.

Rupatadine fumarate was dissolved in macrogol 400. This is the "active solution".

Anhydrous citric acid and anhydrous disodium phosphate were dissolved in purified water and stirred until complete dissolution. This is the "vehicle solution".

The "active solution" and the "vehicle solution" were mixed and homogenized. The preservative was added to the final solution. Finally, purified water was added to the resulting mixture to the desired volume.

| | **Component** | **Amount (mg)** |
|---|---|---|
| Rupatadine fumarate | | 1.28 |
| Macrogol 400 | | 50.00 |
| Anhydrous citric acid | | 4.66 |
| Anhydrous Na₂HPO₄ | | 7.31 |
| Benzalkonium chloride | | 0.20 |
| Purified water qs | | 1.00 mL |
| pH | t₀ | 5.17 |
| % Compound II | 3 months 40°C/75% RH | 0.64 |
| Clarity | 3 months 40°C/75% RH | < I |

### Example 2 to Example 7 Preparation of oral liquid formulations of rupatadine fumarate (1.00 g(rupatadine)/L) with NaOH as pH regulating agent

Liquid formulations of rupatadine fumarate, as detailed in Table 1, were prepared using the following preparation methodology:

Rupatadine fumarate was dissolved in the cosolvent and the preservative, if present, was added to said solution. This is the "active solution".

The pH regulating agent (NaOH 0.1 N) and the sweetening agents, if present, were dissolved in purified water and stirred until complete dissolution. This is the "vehicle solution".

The "active solution" and the "vehicle solution" were mixed and homogenized. The remaining excipients (colorants and flavouring agents), if present, were added to the previous mixture. Then the pH was adjusted to the desired pH when needed by addition of NaOH 0.1 N. Finally, purified water was added to the resulting mixture to the desired volume.

**Table 1**

| **Component** | | **Example 2** | **Example 3** | **Example 4** | **Example 5** | **Example 6** | **Example 7** |
|---|---|---|---|---|---|---|---|
| Rupatadine fumarate | | 0.128 g | 0.128 g | 0.128 g | 0.128 g | 0.100 g | 0.100 g |
| Propylene glycol | | 20.00 g | 20.00 g | 20.00 g | 20.00 g | 20.00 g | 20.00 g |
| Saccharin sodium | | 0.100 g | ---- | ---- | ---- | ---- | ---- |
| NaOH 0.1 N qs | | pH 5 | pH 5 | pH 5 | pH 5 | pH 5.5 | pH 5 |
| Methyl parahydroxybenzoate | | ---- | 0.180 g | ---- | ---- | ---- | ---- |
| Propyl parahydroxybenzoate | | ---- | 0.020 g | ---- | ---- | ---- | ---- |
| 2,4-dichlorobenzylic alcohol | | ---- | ---- | 0.05 g | ---- | ---- | ---- |
| Xanthan gum | | ---- | ---- | ---- | 0.100 g | ---- | ---- |
| Purified water qs | | 100.00 mL | 100.00 mL | 100.00 mL | 100.00 mL | 100.00 mL | 100.00 mL |
| pH | t₀ | 5.02 | 5.01 | 5.00 | 5.00 | 5.60 | 5.00 |
| % | | | | | | | |
| Compound II | 3 months 40°C/75% RH | 0.33 | 0.38 | 0.35 | 0.4 | 0.19‡ | 0.38‡ |
| Clarity | 3 months 40°C/75% RH | <l | <l | <l | NA | NA | NA |

| | | | | | | | |
|---|---|---|---|---|---|---|---|
| NA: Not available. ‡ at 3.5 months. | | | | | | | |

Different sweeteners, colorants, flavouring agents and/or preservatives can be added to the previously described compositions.

### Example 8 to Example 19 Preparation of oral liquid formulations of rupatadine fumarate

### (1.00 g(rupatadine)/L) with a buffer as pH regulating agent

Liquid formulations of rupatadine fumarate, as detailed in Table 2 and Table 3, were prepared using the following preparation methodology:

Rupatadine fumarate was dissolved in the cosolvent and the preservative, if present, was added to said solution. This is the "active solution".

The pH regulating agent (the buffer), and the sweetening agents, if present, were dissolved in purified water and stirred until complete dissolution. This is the "vehicle solution".

The "active solution" and the "vehicle solution" were mixed and homogenized. The remaining excipients (colorants and flavouring agents), if present, were added to the previous mixture. Finally, purified water was added to the resulting mixture to the desired volume.

**Table 2**

| Component | | **Example 8** | **Example 9** | **Example 10** | **Example 11** | **Example 12** | **Example 13** |
|---|---|---|---|---|---|---|---|
| Rupatadine fumarate | | 0.128 g | 0.128 g | 0.128 g | 0.128 g | 0.128 g | 0.128 g |
| Propylene glycol | | 20.00 g | 20.00 g | 20.00 g | 20.00 g | 20.00 g | 20.00 g |
| Saccharin sodium | | ---- | ---- | ---- | ---- | 0.400 g | ---- |
| Sucrose | | ---- | ---- | ---- | ---- | ---- | 50.00 g |
| Anhydrous citric acid | | 0.4656 g | 0.931 g | ---- | ---- | 0.931 g | 0.931g |
| Anhydrous Na₂HPO₄ | | 0.731 g | 1.460 g | ---- | ---- | 1.463 g | 1.463 g |
| Anhydrous KH₂PO₄ | | ---- | ---- | 0.897 g | 1.345 g | ---- | ---- |
| Anhydrous Na₂HPO₄ | | ---- | ---- | 11.35 mg | 17.035 mg | ---- | ---- |
| Purified water qs | | 100.00 mL | 100.00 mL | 100.00 mL | 100.00 mL | 100.00 mL | 100.00 mL |
| pH | t₀ | 5.23 | 5.17 | 4.74 | 4.74 | 5.12 | 5.09 |
| % | | | | | | | |
| Compound II | 3 months 40°C/75% RH | 0.36 | 0.53 | NA | NA | 0.52 | 0.83 |
| Clarity | 3 months 40°C/75%RH | <l | <l | NA | NA | < l‡ | <l‡ |

| | | | | | | | |
|---|---|---|---|---|---|---|---|
| NA: Not available. ^{‡} at 3.5 months | | | | | | | |

Different sweeteners, colorants, flavouring agents and/or preservatives can be added to the previously described compositions.

**Table 3**

| Component | | **Example 14** | **Example 15** | **Example 16** | **Example 17** | **Example 18** | **Example 19** |
|---|---|---|---|---|---|---|---|
| Rupatadine fumarate | | 0.128 g | 0.128 g | 0.128 g | 0.128 g | 0.128 g | 0.128 g |
| Propylene glycol | | 20.00 g | 20.00 g | 20.00 g | 20.00 g | 20.00 g | 20.00 g |
| Saccharin sodium | | ---- | ---- | ---- | ---- | 0.05 g | 0.900 g |
| Sucrose | | ---- | ---- | ---- | ---- | 30.00 g | 50.00 g |
| Anhydrous citric acid | | 0.931 g | 0.931 g | 0.931 g | 0.931 g | 0.466 g | 0.4656 g |
| Anhydrous Na₂HPO₄ | | 1.463 g | 1.463 g | 1.463 g | 1.463 g | 0.731 g | 0.731 g |
| Methyl parahydroxybenzoate | | ---- | ---- | ---- | ---- | 0.100 g | 0.1000g |
| Hypromellose | | 0.400 g | ---- | ---- | ---- | ---- | ---- |
| Sodium carmellose | | ---- | 0.700 g | ---- | ---- | ---- | ---- |
| Quinoline yellow E-104 | | ---- | ---- | ---- | 0.0001 g | 0.0001 g | 0.0001 g |
| Banana flavour | | ---- | ---- | ---- | ---- | 0.25 g | ---- |
| Natural lemon flavour 68%V | | ---- | ---- | 0.400 g | ---- | ---- | 0.400 g |
| Purified water qs | | 100.00 mL | 100.00 mL | 100.00 mL | 100.00 mL | 100.00 mL | 100.00 mL |
| pH | t₀ | 5.16 | 5.16 | 5.16 | 5.15 | 5.18 | 5.19 |
| % Compound II | 3 months 40°C/75% RH | 0.50 | 0.47 | 0.56 | 0.53 | 0.60 | 0.68 |
| Clarity | 3 months 40°C/75% RH | <l‡ | <l‡ | <l‡ | <l‡ | <l | <l‡ |

| | | | | | | | |
|---|---|---|---|---|---|---|---|
| NA: Not available. ‡ at 3.5 months | | | | | | | |

Different sweeteners, colorants, flavouring agents and/or preservatives can be added to the previously described compositions.

### Example 20: Stability of the formulations

As can be shown in the previous tables, the formulations of Example 1 to Example 9 and Example 12 to Example 19 showed good stability properties in the accelerated stability studies as described in *Method 4.*

## Claims

1. A cyclodextrin-free aqueous liquid pharmaceutical composition comprising:
- rupatadine fumarate,
- one or more cosolvents and
- one or more pH regulating agents selected from the group consisting of buffers and bases wherein the composition has a pH between 4 and 6.5, and
wherein the composition is a solution.

2. The pharmaceutical composition according to claim 1 wherein the total amount of cosolvent(s) is from 5 to 50 % of the formulation.

3. The pharmaceutical composition according to any of the preceding claims wherein the cosolvent(s) do not comprise a surfactant.

4. The pharmaceutical composition according to any of the preceding claims wherein the composition comprises one or more cosolvents selected from the group consisting of ethanol, isopropanol, propylene glycol, glycerine, macrogol 300, macrogol 400, dimethylacetamide and pyrrolidone.

5. The pharmaceutical composition according to claim 4 wherein the composition comprises only one cosolvent which is selected from the group consisting of propylene glycol and macrogol 400.

6. The pharmaceutical composition according to claim 5 wherein the one or more pH regulating agents are selected from the group consisting of phosphate buffers, Na₂HPO₄/citric acid, citrate buffers, acetate buffers, ammonium acetate/sodium edetate and NaOH.

7. The pharmaceutical composition according to claims 1 to 6 wherein the pH is between 4.5 and 6.

8. The pharmaceutical composition according to claims 1 to 7 which comprises a sweetener selected from a sugar, an artificial sweetener, and mixtures thereof.

9. The pharmaceutical composition according to any of the preceding claims which comprises rupatadine fumarate at a concentration of 0.5 to 1.5 g(rupatadine)/L.

10. A process to prepare the pharmaceutical composition according to any one of claims 1 to 9 comprising
a) dissolving rupatadine fumarate in the one or more cosolvents to yield a first solution,
b) adding water and the one or more pH regulating agents selected from the group consisting of buffers and bases to the first solution.

11. The pharmaceutical composition according to claims 1 to 9 for use in the treatment of allergic conditions.

12. The pharmaceutical composition for use according to claim 11 wherein the allergic condition is allergic rhinitis or urticaria.

## Patentansprüche

1. Eine Cyclodextrin-freie wässrige pharmazeutische Flüssigformulierung enthaltend:
- Rupatadinfumarat,
- ein Co-Solvens oder mehrere Co-Solvenzien, und
- eine oder mehrere Substanzen zur Einstellung des pH-Wertes ausgewählt aus der Gruppe bestehend aus Puffern und Basen,
wobei die Formulierung einen pH-Wert zwischen 4 und 6,5 aufweist, und wobei die Formulierung eine Lösung ist.

2. Die pharmazeutische Formulierung gemäß Anspruch 1, wobei die Gesamtmenge an Co-Solvens bzw. Co-Solvenzien 5 bis 50 % der Formulierung beträgt.

3. Die pharmazeutische Formulierung gemäß irgend einem der vorherigen Ansprüche, wobei das Co-Solvens oder die Co-Solvenzien keine oberflächenaktive Substanz enthalten.

4. Die pharmazeutische Formulierung gemäß irgend einem der vorherigen Ansprüche, wobei die Formulierung ein Co-Solvens oder mehrere Co-Solvenzien enthält ausgewählt aus der Gruppe bestehend aus Ethanol, Isopropanol, Propylenglykol, Glycerin, Macrogol 300, Macrogol 400, Dimethylacetamid und Pyrrolidon.

5. Die pharmazeutische Formulierung gemäß Anspruch 4, wobei die Formulierung nur ein Co-Solvens enthält, ausgewählt aus der Gruppe bestehend aus Propylenglykol und Macrogol 400.

6. Die pharmazeutische Formulierung gemäß Anspruch 5, wobei die eine Substanz oder die mehreren Substanzen zur Einstellung des pH-Wertes ausgewählt sind aus der Gruppe bestehend aus Phosphatpuffern, Na₂HPO₄/Zitronensäure, Citratpuffern, Acetatpuffern, Ammoniumacetat/Natrium-Edetat und NaOH.

7. Die pharmazeutische Formulierung gemäß Anspruch 1 bis 6, wobei der pH-Wert zwischen 4,5 und 6 liegt.

8. Die pharmazeutische Formulierung gemäß Anspruch 1 bis 7, die ein Süßungsmittel ausgewählt aus einem Zucker, einem künstlichen Süßstoff, und Mischungen davon enthält.

9. Die pharmazeutische Formulierung gemäß irgend einem der vorherigen Ansprüche, die Rupatadinfumarat in einer Konzentration von 0,5 bis 1,5 g (Rupatadin)/L enthält.

10. Ein Verfahren zur Herstellung einer pharmazeutischen Formulierung gemäß irgend einem der Ansprüche 1 bis 9 enthaltend
a) Lösen von Rupatadinfumarat in dem einen Co-Solvens oder in den mehreren Co-Solvenzien, um eine erste Lösung zu erhalten,
b) Zugeben von Wasser und die eine Substanz oder die mehreren Substanzen zur Einstellung des pH-Wertes ausgewählt aus der Gruppe bestehend aus Puffern und Basen zu der ersten Lösung.

11. Die pharmazeutische Formulierung gemäß den Ansprüchen 1 bis 9 zur Verwendung in der Behandlung von allergischen Zuständen.

12. Die pharmazeutische Formulierung zur Verwendung gemäß Anspruch 11, wobei der allergische Zustand allergischer Schnupfen oder Urtikaria ist.

## Revendications

1. Composition pharmaceutique liquide aqueuse sans cyclodextrine comprenant :
- du fumarate de rupatadine,
- un ou plusieurs cosolvants et
- un ou plusieurs agents de régulation du pH sélectionnés dans le groupe constitué par les tampons et les bases
dans laquelle la composition a un pH entre 4 et 6,5, et
dans laquelle la composition est une solution.

2. Composition pharmaceutique selon la revendication 1 dans laquelle la quantité totale de cosolvant(s) va de 5 à 50 % de la formulation.

3. Composition pharmaceutique selon l'une quelconque des revendications précédentes dans laquelle le(s) cosolvant(s) ne comprend(nnent) pas de tensioactif.

4. Composition pharmaceutique selon l'une quelconque des revendications précédentes dans laquelle la composition comprend un ou plusieurs cosolvants sélectionnés dans le groupe constitué par l'éthanol, l'isopropanol, le propylène glycol, la glycérine, le macrogol 300, le macrogol 400, le diméthylacétamide et la pyrrolidone.

5. Composition pharmaceutique selon la revendication 4 dans laquelle la composition ne comprend qu'un seul cosolvant qui est sélectionné dans le groupe constitué par le propylène glycol et le macrogol 400.

6. Composition pharmaceutique selon la revendication 5 dans laquelle le ou les agents de régulation du pH sont sélectionnés dans le groupe constitué par les tampons phosphate, le Na₂HPO₄/acide citrique, les tampons citrate, les tampons acétate, l'acétate d'ammonium/édétate de sodium et le NaOH.

7. Composition pharmaceutique selon les revendications 1 à 6 dans laquelle le pH se situe entre 4,5 et 6.

8. Composition pharmaceutique selon les revendications 1 à 7 qui comprend un édulcorant sélectionné parmi un sucre, un édulcorant artificiel et des mélanges de ceux-ci.

9. Composition pharmaceutique selon l'une quelconque des revendications précédentes qui comprend du fumarate de rupatadine à une concentration de 0,5 à 1,5 g (rupatadine)/I.

10. Procédé de préparation de la composition pharmaceutique selon l'une quelconque des revendications 1 à 9 comprenant
a) la dissolution du fumarate de rupatadine dans le ou les cosolvants pour donner une première solution,
b) l'ajout d'eau et du ou des agents de régulation du pH sélectionnés dans le groupe constitué par les tampons et les bases à la première solution.

11. Composition pharmaceutique selon les revendications 1 à 9 destinée à être utilisée dans le traitement d'affections allergiques.

12. Composition pharmaceutique destinée à être utilisée selon la revendication 11 dans laquelle l'affection allergique est la rhinite allergique ou l'urticaire.
